# EUROPEAN PATENT APPLICATION

(11) **EP 0 943 603 A1**
(43) Date of publication of application: **22.09.1999**
(21) Application number: 99301463.8
(22) Date of filing: 26.02.1999
(51) Int. Cl.: C07C 245/20, C10L 1/00, G01N 33/28, G01N 31/22, C10L 1/22

(54) **Stabilized aquous diazo solutions**

(30) Priority: 18.03.1998 US 40629; 09.09.1998 US 150398
(71) Applicant: MORTON INTERNATIONAL, INC., Chicago, Illinois 60606 (US)
(72) Inventor: Zimin, Al, Sr., Wayne, New Jersey 07470 (US)
(74) Representative: Bankes, Stephen Charles Digby

(57) **Abstract**

A stabilized aqueous solution of a diazo compound has the formula: 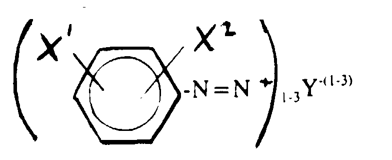 where X¹ is selected from nitro and chloro, X² is selected from nitro, chloro and nothing, and Y is an acid-derived anion. The solution has a pH of about 1.5 or below and a diazo concentration of about 2 wt% or less. The solution is useful as an extractant/developer for petroleum fuel markers.

## Description

The present invention is directed to stabilized aqueous diazo solutions. Such solutions are useful as extractant/developers for petroleum fuel markers such as those described in U.S. Patent No. 4,209,302, the teachings of which are incorporated herein by reference.

### Summary of the Invention

Above-referenced U.S. Patent No. 4,209,302 describes markers for petroleum fuels of certain formulae. Such markers have the formulae: 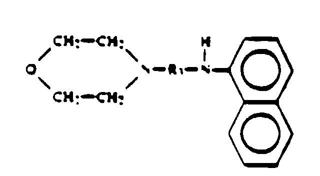 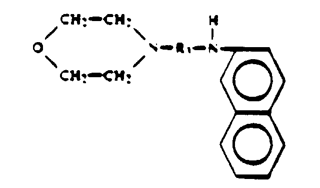 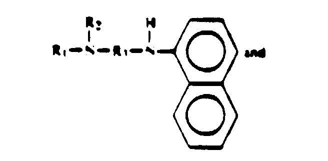 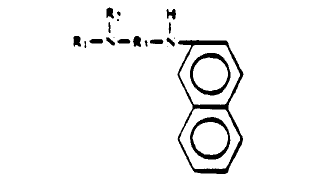 where R₁ and R₂ are hydrogen or alkyl having from one to twenty carbon atoms and R₃ is an alkylene group of from one to eight carbon atoms.

The markers described in U.S. Patent 4,209,302 are identified by extracting the marker from the petroleum fuel with acidic aqueous extractant solution and then adding a stabilized diazo of a compound such as the 2-chlor-4-nitroaniline. U.S. Patent No. 4,209,302 clearly teaches that aqueous solutions of such diazo compounds are unstable. Accordingly, it has been necessary to provide an aqueous acidic extractant separate from a stabilized diazo solution, e.g., the diazo compound in glacial acetic acid. The extraction and color development might be done stepwise, or the two solutions might be mixed just prior to testing the petroleum fuel, but in any case, two solutions have been heretofore provided for field testing of tagged petroleum fuel.

Providing separate extractant and color development is undesirable from the standpoint of the complexity it adds to field testing. Also, as stabilized diazo compounds have some toxicity, it is desirable that they be provided in as dilute form as possible.

Thus, it is a primary object of the present invention to provide a stabilized solution of diazo compounds which act as both an extractant and a color developer.

### Summary of the Invention

In accordance with the present invention there is provided a stabilized aqueous solution of a diazo compound having the formula: 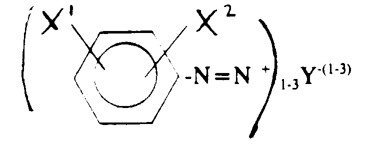 where X¹ is selected from nitro and chloro and X² is selected from nitro, chloro and nothing, provided that one of the Xs is either nitro or chloro and Y is an acid-derived anion,
said solution having a pH of from about 0.5 to about 1.5 or below, preferably between about 1 and about 1.4. said solution having a diazo concentration of between about 0.01 and about 2 wt%, preferably about 1 wt% or less, more preferably about 0.25 wt% or less, most preferably about 0.1 wt% or less.

### Detailed Description of Certain Preferred Embodiments

Herein, and contrary to the teachings of U.S. Patent No. 4,209,302, aqueous solutions of diazo compounds of the above formula have long-term stability, provided that the aqueous solution is very highly acidic (very low pH), and provided the diazo compound is sufficiently dilute. Such stabilized aqueous diazo solutions provide one-solution extraction/color development for petroleum fuel markers, such as those taught in U.S. Patent No. 4,209,302. Because the diazo compounds in such solutions are at very dilute concentrations, they are considered acceptable by many government agencies without specialized precautions taken with respect to their shipping, handling, use and disposal. In fact, useful solutions in accordance with the invention may contain the diazo compound at such low concentrations that the diazo compound not even be listed on safety data sheets.

Particularly suitable compounds of the above-identified formula include, but are not limited to, diazotized 2-chlor-4-nitroaniline, 4-chlor-aniline, and 2-nitro-aniline.

Suitable acids for providing the anion(s) include, but are not limited to propionic acid, phosphoric acid, acetic acid, hydrochloric acid, nitric acid, sulfuric acid, formic acid, methane sulfonic acid and mixtures thereof. Organic acids, such as propionic acid and acetic acid will not alone provide a pH of 1.5 or below, regardless of concentration. Accordingly, while these acids may be present, they must be present in conjunction with at least one other acid. For this reason, also, the stabilized glacial acetic acid formulations described in above-referenced U.S. Patent No. 4,209,302, even if diluted will not provide a pH of 1 or below. Typically, the acid or mixture of acids will be present at between about 10 and about 40 wt% of the composition, most typically between about 20 and about 35 wt%.

Besides the small amount of diazo compound and the acid necessary to provide the low pH, substantially all of the rest of the composition is water. Generally water will be present at at least 40 wt%, more typically at least 50 wt% and above. Depending upon the amount of acid necessary to provide the desired low pH, the water content may be up to about 90 wt% of the solution.

A further advantage of the reagents in accordance with the invention over previous stabilized diazo reagents is that the reagents of the present invention do not freeze when cooled down to 0°C.

The invention will now be described in greater detail by way of specific examples.

### Example 1

A 0.099 wt% solution of diazotized 2-chloro-4-nitroaniline was prepared in a solution of
15 wt% ammonium chloride
15 wt% propionic acid
2.5 wt% phosphoric acid, balance water. (pH 1.10)

The diazo compound proved stable over a period of 34 weeks at room temperature.

### Example 2

A 0.25 wt% solution of diazotized 2-chloro-4-nitroaniline was prepared in a solution of
15 wt% ammonium chloride
15 wt% glacial acetic acid, balance water. (pH 1.25)

The diazo compound proved stable over a period of 34 weeks at room temperature.

### Example 3, comparative

A concentrated solution of diazotized 2-chloro-4-nitroaniline was prepared containing 5 wt% of the dye, 10 wt% sulfuric acid and 85 wt% acetic acid. This was diluted with water to achieve a 0.099 wt% dye solution in water that provided a pH of 1.8 5.

The diazo compound destabilized after a period of 4 days.

This example shows that a primarily acetic acid formulation, even with a small amount of (stronger) sulfuric acid, when diluted, does not provide a sufficiently low pH to stabilize a dilute solution of the dye.

### Example 4. comparative

A 0.25 wt% solution of diazotized 2-chloro-4-nitroaniline was prepared in water (pH = 1.80).

The diazo compound destabilized after a period of 4 days.

## Claims

1. A stabilized aqueous solution of a diazo compound having the formula: 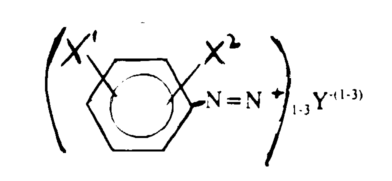 where X¹ is nitro or chloro, X² is selected from nitro, chloro or hydrogen, and Y is an anion or mixture of anions selected from propionate, phosphate, acetate, chloride, nitrate, sulfate, methane sulfonate, formate and sulfonate, said solution having a pH of 0.5 to 1.5, and a diazo concentration of 0.01 to 2 wt%, and containing at least 40 wt% water plus sufficient acid to provide said pH.

2. A solution according to Claim 1 having a pH between 1 and 1.4.

3. A solution according to Claim 1 or Claim 2 having a diazo compound concentration of 1 wt% or less.

4. A solution according to Claim 3 having a diazo compound concentration of 0.25 wt% or less.

5. A solution according to Claim 4 having a diazo compound concentration of 0.1 wt% or less.

6. A solution according to any preceding claim wherein the diazo compound comprises diazotized 2-chloro-4-nitroaniline, 4-chloroaniline or 2-nitroaniline.
